# EUROPEAN PATENT APPLICATION

(11) **EP 4 006 152 A1**
(43) Date of publication of application: **01.06.2022**
(21) Application number: 20848232.3
(22) Date of filing: 26.06.2020
(51) Int. Cl.: C12N 15/113, C12N 15/63, C12N 15/75, C12N 1/21

(54) **TANDEM PROMOTER, BACTERIUM OF THE GENUS BACILLUS PRODUCING A TARGET PRODUCT CONTAINING SAID PROMOTER, AND METHOD OF PRODUCING THE TARGET PRODUCT**

(30) Priority: 26.07.2019 RU 2019123608
(71) Applicant: Obshchestvo s Ogranichennoi Otvetstvennostyu "Centr Transfera Biotechnologiy Oka-Biotech", Moscow, 119421 (RU)
(72) Inventor: MIRONOV, Aleksandr Sergeevich, Moscow, 105094 (RU); ERRAIS, Lopes Lyubov, Moscow, 105094 (RU); BAYEVA, Olga Viktorovna, Moscow, 119333 (RU); BATTALOVA, Irina Yunosovna, Tuchkovo, 143132 (RU); BROVKIN, Aleksey Nokolaevich, Moscow, 117420 (RU); CMIRNOV, Petr Vladimirovich, Moscow, 117042 (RU); RYKOV, Sergey Viktorovich, Shkolnoe, 352621 (RU); URCHENKO, Uliay Valerevna, Moscow, 115597 (RU); BEBUROV, Mihail Urevich, Moscow, 119607 (RU); KALUJSKIY, Vasiliy Evgenevich, Moscow, 119421 (RU)
(74) Representative: Osmans, Voldemars
(86) International application number: PCT/RU2020/000312
(87) International publication number: WO 2021/020996

(57) **Abstract**

The present invention relates to a tandem promoter P_{rpsF-gsiB}, functioning in a bacterium of the genus *Bacillus,* to a host cell that is a bacterium of the genus *Bacillus* producing a target product, containing said promoter, and to a method for producing the target product using said host cell. The use of said promoter makes it possible to obtain an optimal expression profile for the target gene during cultivation of the host cell and to effectively produce target products using said host cell.

## Description

### TECHNICAL FIELD

The present invention relates to biotechnology, in particular, to a tandem promoter functioning in a bacterium of the *Bacillus* genus and allowing said bacterium to express target genes used for the production of target products. The present invention also relates to a bacterium of the genus *Bacillus* which is a producer of the target product, wherein the bacterium comprises said promoter controlling expression of the target genes, and to a method for producing the target product using said bacterium.

### BACKGROUND ART

Bacteria of the *Bacillus* genus are well known as host cells for producing various target products.

Usually, the maximal expression of target genes comprised by a *Bacillus* cell and required for producing target products is achieved via amplification of an expression cassette comprising a single promoter operably linked to a target gene of interest and an amplifiable selectable marker gene, e.g., an antibiotic resistance marker, in the cell chromosome. This amplification results in several copies of the expression cassette with the selectable marker gene in the chromosome.

Such *Bacillus* bacteria host cells, however, are not always characterized by properties required for their commercial use and have some disadvantages associated with the above approach. For instance, it may not be possible to achieve saturating levels of mRNA by amplification of genes driven by a single promoter. Furthermore, removal of the selectable marker genes without loss of expression cassettes may be technically unfeasible.

The maximal expression of target genes required for obtaining target products can also be ensured by using strong promoters, inducible promoters, multiple promoters or tandem promoters, as well as by stabilizing mRNA.

US Patent No. 5,955,310 describes the use, in *B. subtilis* bacteria, of an expression cassette comprising the P_{amyQ-cryIIIA} tandem promoter operably linked to a single copy of the nucleic acid encoding the target gene, and a sequence downstream of the promoter and upstream of a target gene, said sequence stabilizing the mRNA. This document, however, does not contain information about the activity profile of the claimed promoter during bacterial cultivation.

Patent application WO 201004625 A1 describes the use of the P_{rpsB-subC} tandem promoter in *B. licheniformis* and *B. pumilus* bacteria, and shows that this use allows to increase protease 1 and protease 2 production by 24% and 38%, respectively, compared to the use of a single P_{subC} promoter.This document, however, contains no information about the activity profile of the claimed promoter during bacterial cultivation.

Patent application CN 105779444 (A) describes the use of P_{43-HpaII-SPO-I} and P_{43-HpaII-SPO-I-cryIIIA} tandem promoters in bacteria of the genus *Bacillus.* It was shown that the first promoter allows to increase the alkaline protease production by 70% compared to the total yield in bacteria comprising single promoters, while the use of the second promoter makes it possible to increase the alkaline protease production by 50% compared to the total yield in bacteria comprising single promoters.This document, however, contains no information on the activity profile of the claimed promoters during bacterial cultivation.

### SUMMARY OF INVENTION

A technical object of the present invention is development of a more productive method for microbial synthesis of target products using bacteria of the genus *Bacillus,* and expanding the toolkit of artificial promoters that are functional in bacteria of the genus *Bacillus* and have improved properties required for production of target products.

This object has been achieved by constructing a P_{rpsF-gsiB} tandem promoter with improved properties required for production of target products. Expression of a target gene placed under control of said P_{rpsF-gsiB} tandem promoter is increasing during cultivation of the *Bacillus* bacterium producing a target protein and reaches its maximum at the late stationary phase, thereby ensuring the most efficient target gene expression profile and the most efficient production of the target product. A method for production of target products using said bacteria is easier and more technology-savvy, since the use of the P_{rpsF-gsiB} tandem promoter does not require adding an expression inducer during bacterial cultivation as opposed to the use of, e.g. inducible promoters.

In one of the aspects, the present invention relates to a tandem promoter operable in a bacterium of the genus *Bacillus* and containing the nucleotide sequences of the *rpsF* gene promoter and the *gsiB* gene promoter, wherein the *rpsF* gene promoter is located upstream of the *gsiB* gene promoter. The P_{rpsF} promoter is one of the strongest promoters in the *B. subtilis* genome and provides the highest transcription level in the logarithmic phase of bacterial growth. The *gsiB* gene transcription initiation involves the alternative sigma subunit (σB) and reaches its maximum at the stationary growth phase. In another aspect, the present invention relates to the above-described tandem promoter comprising the nucleotide sequences of the *rpsF* gene promoter and the *gsiB* gene promoter from *Bacillus subtilis.*

In the next aspect, the present invention relates to the above-described tandem promoter comprising the nucleotide sequence set forth in the Sequence Listing as SEQ ID NO: 1.

In another aspect, the present invention relates to a bacterium of the genus *Bacillus* producing a target product and comprising the above-described tandem promoter operably linked to a nucleic acid sequence encoding a polypeptide which is a target product or a polypeptide required for producing the target product.

In another aspect, the present invention relates to the above-described bacterium selected from the group, comprising *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* and *Bacillus thuringiensis.*

In one of the aspects, the present invention further relates to the above-described *Bacillus subtilis* bacterium.

In a further aspect, the present invention relates to the above-described bacterium wherein the nucleic acid sequence, encoding the polypeptide which is the target product or the polypeptide required for producing the target product, is a heterologous sequence.

In another aspect, the present invention relates to the above-described bacterium producing a target product, selected from the group consisting of amino acids, polypeptides, carbohydrates, polysaccharides, nucleic acids, and derivatives thereof.

In the next aspect, the present invention relates to a method for producing a target product, said method comprising culturing said bacterium of the *Bacillus* genus in a nutrient medium useful for producing the target product, and isolating the target product from the culture broth.

In a further aspect, the present invention relates to the method for producing a target product, wherein said target product is selected from the group consisting of amino acids, polypeptides, carbohydrates, polysaccharides, nucleic acids, and derivatives thereof.

### BRIEF DESCRIPTION OF DRAWINGS

Figure 1 shows the nucleotide sequence of the P_{rpsF} promoter. Capital letters denote -35 and -10 promoter regions; the ribosome-binding site is underlined. The primer sequences are shown in bold.
Figure 2 shows the nucleotide sequence of the P_{gsiB} promoter. Capital letters denote -35 and -10 promoter regions; the ribosome-binding site is underlined. The primer sequences are shown in bold.
Figure 3 shows structure of the plasmid pDG268.
Figure 4 shows the nucleotide sequence of the P_{rpsF-gsiB} hybrid promoter. Capital letters denote -35 and -10 promoter regions; the ribosome-binding site is underlined. Transcription start sites are designated +1. The junction of P_{rpsF} and P_{gsiB} promoters is shown by an arrow.
Figure 5 shows comparative data on the efficiency of the P_{rpsF-gsiB} hybrid promoter and the single P_{rpsF} promoter during *B. subtilis* fermentation.

### DESCRIPTION OF EMBODIMENTS

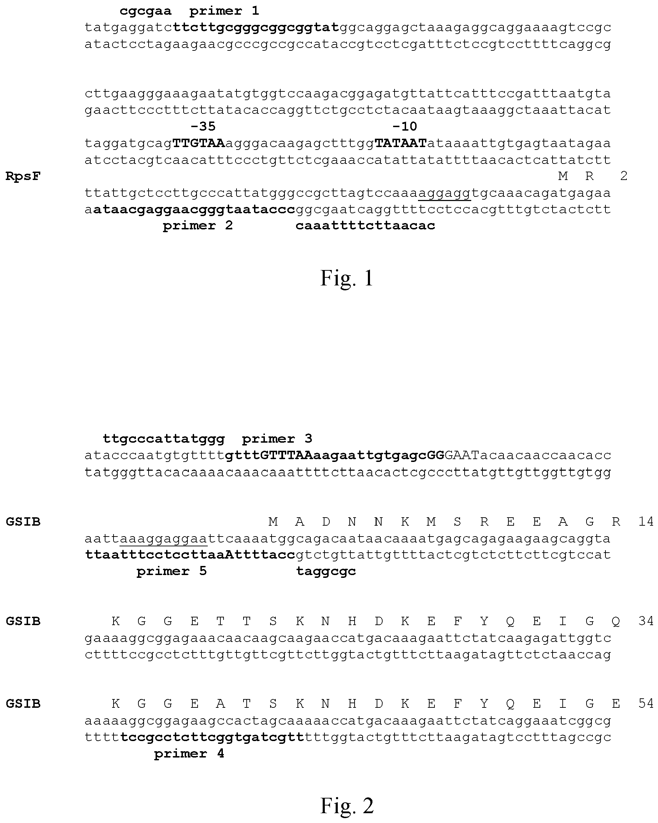

The P_{rpsF-gsiB} tandem promoter according to the present invention is a fusion of the *rpsF* gene promoter, where the *rpsF* gene encodes the synthesis of the S6 ribosomal protein, and the *gsiB* gene promoter responsible for synthesis of the general cellular stress protein (PgsiB), wherein the *rpsF* gene promoter is located upstream of the *gsiB* gene promoter. The P_{rpsF} promoter comprises a region similar to the -35 consensus sequence (TTGTAA) and the consensus sequence of the -10 region (TATAAT). The P_{rpsF} promoter in one of the strongest promoters in the *Bacillus* bacterial genome, in particular, *B. subtilis* genome; it provides the maximal transcription level in the logarithmic phase of bacterial growth. This promoter transcription initiation involves the RNA polymerase vegetative sigma subunit (σ^{A}). The nucleotide sequence of the *B. subtilis rpsF* gene is available in the GenBank database as GeneID: 937919 (nucleotides 4199445 to 4199732 in NC_000964.3). The *B. subtilis* P_{rpsF} promoter nucleotide sequence is shown in Figure 1. The *gsiB* gene transcription initiation involves the alternative RNA polymerase subunit σ^{B} and reaches its maximum in the stationary phase of bacteria growth. The nucleotide sequence of the *B. subtilis gsiB* gene is available in the GenBank database as GeneID: 938235 (nucleotides 494506 to 494877 in NC_000964.3). The *B. subtilis* P_{rpsF} promoter nucleotide sequence is shown in Figure 2.

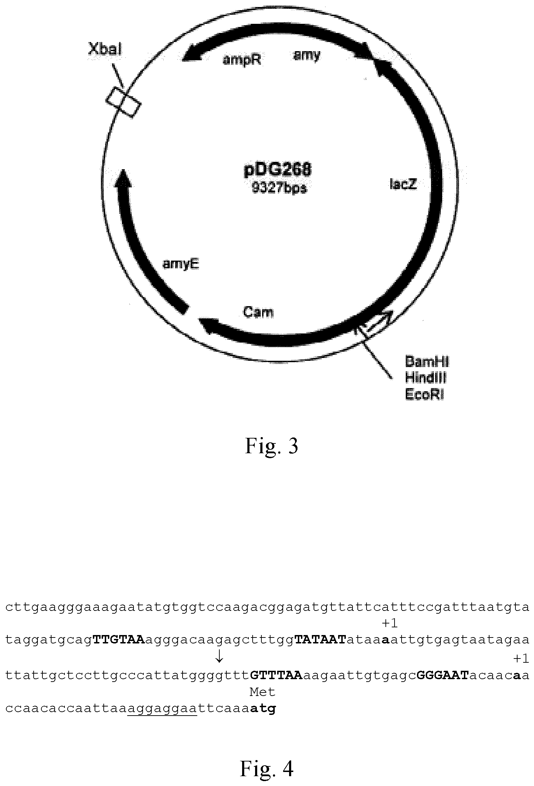

The nucleotide sequence of the P_{rpsF-gsiB} tandem promoter according to the present invention is represented in Figure 4 and set forth in the Sequence Listing as SEQ ID NO: 1.

The terms "P_{rpsF-gsiB} tandem promoter is operably linked" to a target gene, or "a gene is under control of the P_{rpsF-gsiB} hybrid tandem promoter" mean that the naturally occurring promoters of these genes have been replaced with the P_{rpsF-gsiB} promoter.

As described herein, in one of the illustrative embodiments, the P_{rpsF-gsiB} tandem promoter is operably linked to the reporter *lacZ* gene encoding β-galactosidase. In another illustrative embodiment, by way of non-limiting example only and to study the expression profile of genes placed under control of the P_{rpsF-gsiB} tandem promoter during cultivation of the bacteria having this tandem promoter, said tandem promoter is operably linked to a nucleic acid sequence associated with the target protein production. However, a person skilled in the art would readily appreciate that said P_{rpsF-gsiB} tandem promoter can be operably linked to any gene, gene fusion, or operon associated with production of a target product, and would provide an expression profile of this gene, gene fusion, or operon that is almost the same as that of the reporter *lacZ* gene as described herein.

The term "bacterium of the genus *Bacillus"* means a bacterium that belongs to the *Bacillus* genus in accordance with the classification known to those skilled in the art of microbiology.

For instance, the genus *Bacillus* include, but not limited to the following bacteria: *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* and *Bacillus thuringiensis.* Use of bacteria of the *Bacillus subtilis* species is preferable.

The genus *Bacillus* is known to include over 200 bacterial species, and the most practically important of them not only share common features with regard to morphology, physiology and cell culture, but also show certain similarities in their genome organization and gene expression mechanisms. For example, direct experiments have shown that, for example, the *B. subtilis aprE* extracellular proteinase gene promoter is able to initiate synthesis of a reporter protein in the cells of various bacilli species, sometimes even to a greater extent compared to the *B. subtilis* cells (Genome Biology, 5, 77, 2004).

The term "heterologous gene" means that said gene has been isolated from the chromosome of the organism other than the host cell, for instance, other than the bacteria of the *Bacillus* genus, in particular *B. subtilis.*

The term "target product" means any chemical compound of interest that is produced by a bacterium and accumulated within cells in a soluble or other form (e.g., as inclusion bodies), or accumulated in the nutrient medium where the bacterium is cultivated. The target product includes but is not limited to compounds selected from the group consisting of amino acids, polypeptides, carbohydrates, polysaccharides, nucleic acids, and derivatives thereof.

Methods according to the present invention include a method for producing a target product comprising the steps of culturing the bacterium of the present invention in a nutrient medium for production and accumulation of the target product, and subsequent isolation thereof.

According to the present invention, cultivation, collection and purification of the useful metabolite from the culture broth or the like can be performed by a method similar to conventional culture methods where the target product is produced using a microorganism. The nutrient medium used for cultivation can be either synthetic or natural provided that said medium contains carbon and nitrogen sources, mineral additives, and, where necessary, the appropriate amounts of nutritive supplements required for microbial growth. Carbon sources include various carbohydrates such as glucose and sucrose, and various organic acids. Various ammonium salts such as ammonium sulfate, and other nitrogenous compounds such as ammonia, amines, natural nitrogen sources such as peptone, soybean hydrolysate and microbial enzymatic hydrolysate can be used as nitrogen sources. Potassium monophosphate, magnesium sulfate, sodium chloride, ferrous sulfate, manganese sulfate, calcium chloride, and similar substances can be used as mineral additives (Anagnostopoulos C., Spizizen J. Requirement for transformation in Bacillus subtilis // J. Bacteriol. 1961. V. 81, P. 7411. In); C. R. Harwood and S. M. Cutting (ed.) Molecular biological methods for Bacillus// Wiley, Chichester, United Kingdom, 1990).

The cultivation is preferably performed under aerobic conditions such as stirring or aerated fermentation at temperatures from 20 °C to 40 °C, preferably from 30 °C to 38 °C. The pH of the nutrient medium is maintained in the range from 5 to 9, preferably from 6.5 to 7.2. The pH of the medium can be adjusted by addition of ammonia, calcium carbonate, various acids, bases, and buffers. Usually, the target product accumulates in the culture broth after 1 to 5 days of cultivation.

When the cultivation is completed, solid residues such as cells can be removed from the culture broth by centrifugation or membrane filtration, and then the target product can be collected and purified by chromatography, concentration, and/or crystallization, fractional salt precipitation, precipitation with organic solvents, etc., and any combination of the above methods.

The present invention will be described in more detail with reference to the examples.

### EXAMPLES

The following examples are provided for the purpose of clarifying the essence of the present invention and not intended to limit the scope of the present invention defined by the claims.

### Example 1. Construction of the hybrid P_{rpsF-gsiB} promoter

The P_{rpsF-gsiB} hybrid promoter was constructed in several steps. A 193-bp fragment of the P_{rpsF} promoter was initially PCR-amplified from *B. subtilis* chromosomal DNA using primer 1 (SEQ ID NO: 2) comprising an *Eco*RI restriction site and primer 2 (SEQ ID NO: 3) comprising at the 5'-end a sequence that is complementary to the *gsiB* gene promoter region. The P_{rpsF} promoter nucleotide sequence with indicated locations of primers and sequences thereof is shown in Figure 1.

PCR amplification was carried out in a 50-µL incubation mixture containing 5 ng of *B. subtilis* chromosomal DNA as a template, 20 pmol of each of primers 1 and 2, 1xPCR buffer, a mixture of deoxyribonucleoside triphosphates (dNTPs) (0.5 mM each), and 2 U of a thermostable *Taq* polymerase. The reactions were carried out in My Cycler BioRad thermal cycler programmed for 1 cycle of 95 °C for 5 min; 30 cycles of 95 °C for 30 s, 56 °C for 30 s and 72 °C for 30 s each; and 1 cycle of 72 °C for 5 min. The products were analyzed by gel electrophoresis in 1.2% agarose in 1xTAE (Sambrook J., Fritsch E.F., Maniatis T. Molecular cloning: a laboratory manual. 2nd ed. N.Y.: Cold Spring Harbor Lab., 1989). The expected fragment was about 190 bp in size.

At the next step, the 184-bp fragment of the *gsiB* gene promoter was amplified using primer 3 (SEQ ID NO: 4) comprising at the 5'-end a sequence that is complementary to the P_{rpsF} promoter region, and primer 4 (SEQ ID NO: 5) comprising at the 5'-end a sequence that is complementary to the P_{rpsF} promoter region and primer 4. The P_{gsiB} promoter nucleotide sequence with indicated locations of primers and sequences thereof is shown in Figure 2.

The PCR amplification was carried out in a 50-µL incubation mixture with 5 ng of *B. subtilis* chromosomal DNA as a template, 20 pmol of primer 3 and primer 4 each, 1xPCR buffer, a dNTPs mixture (0.5 mM each), and 2 U of a thermostable *Taq* polymerase. The reactions were carried out in My Cycler BioRad thermal cycler programmed for 1 cycle of 95 °C for 5 min; 30 cycles of 95 °C for 30 s, 56 °C for 30 s, 72 °C for 30 s each; and 1 cycle of 72 °C for 5 min. The products were analyzed by gel-electrophoresis in 1.2 agarose in 1xTAE. The expected fragment was about 200 bp in size.

Then these two obtained fragments were aligned by annealing to each other by PCR using the following mode: PCR amplification in a 50-µL mixture comprising 5 ng of each DNA fragment, 1xPCR buffer, a dNTPs mixture (0.5 mM each), and 2 U of thermostable *Taq* polymerase. The reactions were carried out in a thermal cycler programmed for 1 cycle of 95 °C for 5 min; 10 cycles of 95 °C for 30 s, 52 °C for 30 s and 72 °C for 2 min each; and 1 cycle of 72 °C for 5 min. Next, primer 1 (SEQ ID NO: 2) comprising *an Eco*RI restriction site, and primer 5 (SEQ ID NO: 6) comprising a *Bam*HI restriction site were added to the reaction mixture. PCR was performed in the following mode: 25 cycles of 95 °C for 30 s, 56 °C for 30 s, 72 °C for 30 s each; and 1 cycle of 72 °C for 5 min. The products were analyzed by gel-electrophoresis in 1.2% agarose and 1xTAE. The expected fragment was about 255 bp in size.

The obtained combined 255 bp PCR fragment was treated with the *Eco*RI and *Bam*HI restriction endonucleases and cloned into appropriate sites of the pDG268 plasmid (Cell 111: 747-756, 2002), which includes a cassette comprising a cloning polylinker, the *lacZ* reporter gene without natural promoter, and the chloramphenicol resistance gene (Cm^{R}) (Figure 3). The cassette is flanked with *amyE* gene fragments, which allows to integrate the vector comprising the cloned fragment into the *amyE* locus on the *B. subtilis* chromosome. The obtained plasmid, named pDG268-P, was used for the transformation of the competent *E. coli* TG1 cells. The transformants were selected on LB medium containing 100 µg/mL ampicillin. Plasmid DNA was purified on agarose gel and using columns (GFX PCR DNA and Gel Band Purification Kit, Illustra) or DNA Gel Extraction Kit beads (Fermentas). The sequence of the DNA insert was confirmed by DNA sequencing with primer 6 (SEQ ID NO: 7, forward primer) and primer 7 (SEQ ID NO: 8, reverse primer).

At the final step, the obtained plasmid named pDG268-P comprising the P*_{rpsF-gsiB}* hybrid promoter was integrated into the *B. subtilis amyE* chromosomal locus. For this purpose, prior to the *B. subtilis* cells transformation, pDG268-P plasmid DNA was linearized using the *Xba*I restriction endonuclease to ensure the integration into the chromosome by double crossover instead of simple crossover. The competent *B. subtilis* strain 168 cells were transformed with the *Xba*I-treated pDG268-P plasmid DNA, and the transformants were selected on LB medium comprising 10 µg/mL chloramphenicol. To confirm integration of the plasmid into the *amyE* chromosomal locus, an indicator medium containing amylopectinase (0.2%) was used.

Thus, the performed manipulations yielded the plasmid pDG268-P comprising the P_{rpsF-gsiB} hybrid promoter and the adjacent reporter *lacZ* gene, which allows to test activity of said hybrid promoter under fermentation conditions. The P_{rpsF-gsiB} hybrid promoter nucleotide sequence is shown in Figure 4.

As is seen from Figure 4, the hybrid promoter contains two -35 and two -10 regions, in addition to the two transcription start sites, which, as the authors of the present invention assume without intending to be limited by any theory, can be used alternately at different stages of culture growth and provide optimal expression of genes controlled by said promoter.

### Example 2. Assessing efficiency of the P_{rpsF-gsiB} hybrid tandem promoter

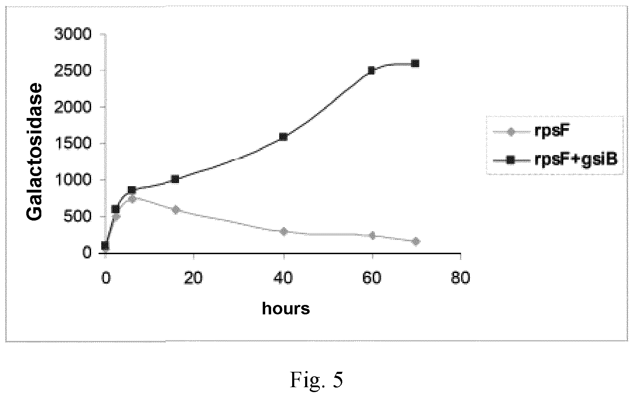

To determine efficiency of the P_{rpsF-gsiB} hybrid tandem promoter, the pDG268-P plasmid prepared at the previous step and comprising the transcription fusion of the P_{rpsF-gsiB} promoter and the *lacZ* reporter gene, was used to transform the *B. subtilis* strain 168 cells, and the resulting transformants were analyzed for the β-galactosidase activity during fermentation in complete LB medium. The concomitantly engineered variant of transcription fusion of the *lacZ* gene and single P_{rpsF} promoter was used as a control. Samples for measuring β-galactosidase activity were taken 2, 5, 6, 16, 40, 60 and 70 h after the fermentation start. The results of the β-galactosidase activity measurements are represented in Figure 5.

As shown in Figure 5, efficiency of the tested promoters is roughly the same until the early stationary growth phase; the activity of the single P_{rpsF} promoter, however, starts to decrease from the 16^{th} hour of fermentation, and drops by almost an order of magnitude by the 60^{th} hour. At the same time, activity of the P_{rpsF-gsiB} hybrid tandem promoter continues to increase during fermentation and reaches its maximum at the late stationary growth phase. Thus, the P_{rpsF-gsiB} hybrid promoter constructed according to the present invention allows to provide an optimal target gene expression profile during bacterial cultivation, and is very promising for enhancing expression of target genes used for the production of useful metabolites.

### Example 3. Preparation of AM2640 genetic construct by introducing a fusion of hasA gene from S. equisimilis and tuaD gene from B. subtilis under control of the hybrid tandem P_{rpsF-gsiB} promoter into chromosome of recipient Bacillus subtilis strain 168

Genes involved in the biosynthesis of the saccharide precursors to produce hyaluronic acid are known to comprise, in particular, the *tuaD* gene of *B. subtilis* encoding UDP-glucoso-6-dehydrogenase (EC 1.1.1.22). In the *Streptococcus pyogenes* chromosome, the hyaluronan synthase operon consists of the three genes, *hasA, hasB,* and *hasC,* which encode hyaluronate synthase, UDP-glucose dehydrogenase, and UDP-glucose pyrophosphorylase, respectively (Proc. Natl. Acad. Sci. USA. 98, 4658-4663, 2001). International publication WO 99/51265 describes the nucleic acid segment comprising the coding region of the *Streptococcus equisimilis* hyaluronate synthase.

The fusion of *hasA* gene from *S. equisimilis* and *tuaD* gene of *B. subtilis* under the P_{rpsF-gsiB} hybrid tandem promoter control was constructed in several steps. First, the *hasA* gene was chemically synthesized based on the nucleotide sequence of *hasA* gene from *S. equisimilis* and cloned into pUC57 vector. The *hasA* gene (1250 bp) was PCR-amplified from the resulting plasmid DNA using primer 8 (SEQ ID NO: 9) comprising a *Bam*HI recognition site and primer 9 (SEQ ID NO: 10) comprising at the 5'-end a sequence that is complementary to the N-terminal region of the *tuaD* gene of *B. subtilis.*

PCR amplification was carried out in a 50-µL incubation mixture containing 3 ng of pUC57 plasmid DNA as a template, 20 pmol of primer 8 (SEQ ID NO: 9) and primer 9 (SEQ ID NO: 10) each, 1xPCR buffer, a dNTPs mixture (0.5 mM each), and 2 U of a thermostable *Taq* polymerase. The reactions were carried out in My Cycler BioRad thermal cycler programmed for 1 cycle of 95 °C for 5 min; 30 cycles of 95 °C for 30 s, 58 °C for 30 s, and 72 °C for 2 min each; and 1 cycle of 72 °C for 5 min. The products were analyzed by gel-electrophoresis in 1.2% agarose in 1xTAE. The expected fragment was about 1270 bp in size.

At the next step, the *tuaD* gene was PCR-amplified from the *B. subtilis* chromosomal DNA using primer 10 (SEQ ID NO: 11) comprising at the 5'-end a sequence that is complementary to the C-terminal end of *hasA* gene from *S. equisimilis,* and primer 11 (SEQ ID NO: 12) comprising a *Not*I recognition site.

PCR amplification was carried out in a 50-µL incubation mixture containing 5 ng of *B. subtilis* chromosomal DNA as a template, 20 pmol of primer 10 (SEQ ID NO: 11) and primer 11 (SEQ ID NO: 12) each, 1xPCR buffer, a dNTPs mixture (0.5 mM each), and 2 U of a thermostable *Taq* polymerase. The reactions were carried out in My Cycler BioRad thermal cycler programmed for 1 cycle of 95 °C for 5 min; 30 cycles of 95 °C for 30 s, 58 °C for 30 s, and 72 °C for 2 min each; and 1 cycle of 72 °C for 5 min. The products were analyzed by gel-electrophoresis in 1.2% agarose in 1xTAE. The expected fragment was about 1450 bp in size.

Then, the DNA fragments obtained using primer pairs 8/9 and 10/11 were aligned by annealing to each other by PCR using the following mode: PCR amplification in a 50-µL incubation mixture containing 5 ng DNA of the fragments prepared using primer pairs 8/9 and 10/11 each, 1xPCR buffer, a dNTPs mixture (0.5 mM each), and 2 U of a thermostable *Taq* polymerase. The reactions were carried out in My Cycler BioRad thermal cycler programmed for 1 cycle of 95 °C for 5 min; 10 cycles of 95 °C for 30 s, 52 °C for 30 s, and 72 °C for 2 min each; and 1 cycle of 72 °C for 5 min. Next, primer 8 (SEQ ID NO: 9) comprising a *Bam*HI recognition site and primer 11 (SEQ ID NO: 12) comprising a *Not*I recognition site were added to the reaction mixture, and PCR was carried out in the following mode: 25 cycles of 95 °C for 30 s, 56 °C for 30 s and 72 °C for 2.5 min each; and 1 cycle of 72 °C for 5 min. The products were analyzed by gel-electrophoresis in 1.2% agarose in 1xTAE. The expected fragment was about 2,700 bp in size.

The resulting 2,700-bp PCR fragment was treated with the *Bam*HI and *Not*I restriction endonucleases and cloned into the corresponding sites of the previously prepared pDG268-P plasmid comprising the P_{rpsF-gsiB} hybrid tandem promoter. The resulting plasmid, named pDG268-PHT, was used to transform the competent *E. coli* TG1 strain cells. The transformants were selected on LB medium containing 100 µg/mL ampicillin. Plasmid DNA was purified in agarose gel and using columns (GFX PCR DNA and Gel Band Purification Kit, Illustra), or Gel Extraction kit beads (Fermentas). The DNA insert sequence was confirmed by DNA sequencing using primer 12 (SEQ ID NO: 13, forward) and primer 13 (SEQ ID NO: 14, reverse).

At the final step, the produced pDG268-PHT plasmid was integrated into the *B*. *subtilis amyE* chromosomal locus. For this purpose, the pDG268-PHT plasmid DNA was linearized prior to the transformation of *B. subtilis* cells using the *Xba*I restriction endonuclease to ensure integration into the chromosome by double crossover instead of simple crossover. The competent *B. subtilis* strain 168 cells were transformed with the *Xba*I-treated DNA of the pDG268-PHT plasmid, and the transformants were selected on LB medium containing 10 µg/mL chloramphenicol. To confirm integration of the plasmid into the *amyE* chromosomal locus, an indicator medium containing amylopectinase (0.2%) was used.

Thus, the performed manipulations yielded a *B. subtilis-based* construct which comprises in its chromosome the fusion of *hasA* gene from *S. equisimilis* and *tuaD* gene from *B. subtilis* under control of the P_{rpsF-gsiB} hybrid tandem promoter.

### Example 4. Hyaluronic acid synthesis by the AM2640 construct during cultivation

The genetic construct prepared according to Example 3 and comprising the fusion of *hasA* gene from *S. equisimilis* with the *tuaD* gene from *B. subtilis* under control of the P_{rpsF-gsiB} hybrid tandem promoter was tested for capability to produce hyaluronic acid during cultivation in a laboratory 3 L fermenter in a fermentation medium. The results are shown in Table 1.

**Table 1. Kinetics of hyaluronic acid accumulation in culture broth of the obtained genetic construct AM2640**

| **Genetic construct** | **Genotype** | **Fermentation time (hours)** | |
|---|---|---|---|
| | | **24** | **48** |
| | | **Hyaluronic acid accumulation (g/L)** | |
| ***B. subtilis* 168 (original strain)** | **Wild type** | **<0.01** | **<0.01** |
| **AM2640** | *amyE*:: P*_{rpsF-gsiB}-hasA-tuaD* | **1.5** | **1.8** |

As shown in Table 1, the resulting genetic construct demonstrated a much higher level of the hyaluronic acid accumulation compared to the original *B. subtilis* 168 strain.

Although the present invention has been described above with reference to the preferred embodiments, a person skilled in the art would readily appreciate that various substitutions can be made and various equivalents can be used without departing from the scope of the present invention. All documents cited herein are the part of the present application and are incorporated herein by reference.

Sequence                                    SEQUENCE LISTING

<110>  OBSHCHESTVO S OGRANICHENNOI OTVETSTVENNOSTYU "CENTR
       TRANSFERA BIOTECHNOLOGIY OKA-BIOTEH"

<120>  TANDEM PROMOTER, BACTERIUM OF THE GENUS  BACILLUS PRODUCING A
       TARGET PRODUCT CONTAINING SAID PROMOTER, AND METHOD OF PRODUCING
       THE TARGET PRODUCT

<130>

<140>  PCT/RU2020/000312
<141>  2020-06-26

<150>  RU 2019123608
<151>  2019-07-26

<160>  14

<170>  PatentIn version 3.5

<210>  1
<211>  210
<212>  DNA
<213>  Artificial Sequence

<220>
<223>  Tandem promoter PrpsF-gsiB

<400>  1
cttgaaggga aagaatatgt ggtccaagac ggagatgtta ttcatttccg atttaatgta      60

taggatgcag ttgtaaaggg acaagagctt tggtataata taaaattgtg agtaatagaa     120

ttattgctcc ttgcccatta tggggtttgt ttaaaagaat tgtgagcggg aatacaacaa     180

caacaccaat taaaggagga attcaaaatg                                      210


<210>  2
<211>  25
<212>  DNA
<213>  Artificial Sequence

<220>
<223>  Primer 1

<400>  2
cgcgaattct tgcgggcggc ggtat                                            25


<210>  3
<211>  39
<212>  DNA
<213>  Artificial Sequence

<220>
<223>  Primer 2

<400>  3
cacaattctt ttaaacccca taatgggcaa ggagcaata                             39

<210>  4
<211>  39
<212>  DNA
<213>  Artificial Sequence

<220>
<223>  Primer 3

<400>  4
ttgcccatta tggggtttgt ttaaaagaat tgtgagcgg                                39


<210>  5
<211>  21
<212>  DNA
<213>  Artificial Sequence

<220>
<223>  Primer 4

<400>  5
ttgctagtgg cttctccgcc t                                                   21


<210>  6
<211>  31
<212>  DNA
<213>  Artificial Sequence

<220>
<223>  Primer 5

<400>  6
cgcggatcca ttttaaattc ctcctttaat t                                        31


<210>  7
<211>  18
<212>  DNA
<213>  Artificial Sequence

<220>
<223>  Primer 6

<400>  7
taagggtaac tattgccg                                                       18


<210>  8
<211>  20
<212>  DNA
<213>  Artificial Sequence

<220>
<223>  Primer 7

<400>  8
taagttgggt aacgccaggg                                                     20


<210>  9
<211>  37
<212>  DNA

<213>  Artificial Sequence

<220>
<223>  Primer 8

<400>  9
cgcggatcct aaatgagaac attaaaaaac ctcataa                                37


<210>  10
<211>  46
<212>  DNA
<213>  Artificial Sequence

<220>
<223>  Primer 9

<400>  10
ttcattttga attcctcctt tttataataa ttttttacgt gttccc                      46


<210>  11
<211>  46
<212>  DNA
<213>  Artificial Sequence

<220>
<223>  Primer 10

<400>  11
tataaaaagg aggaattcaa aatgaaaaaa atagctgtca ttggaa                      46


<210>  12
<211>  27
<212>  DNA
<213>  Artificial Sequence

<220>
<223>  Primer 11

<400>  12
cccgcggccg ccgagcctag cagccgg                                          27


<210>  13
<211>  18
<212>  DNA
<213>  Artificial Sequence

<220>
<223>  Primer 12

<400>  13
taagggtaac tattgccg                                                    18


<210>  14
<211>  20
<212>  DNA
<213>  Artificial Sequence

<220>

<223>  Primer 13

<400>  14
taagttgggt aacgccaggg                                                    20

## Claims

1. A tandem promoter operable in a host cell of the genus *Bacillus* and comprising nucleotide sequences of *rpsF* gene promoter and *gsiB* gene promoter derived from *Bacillus subtilis,* wherein the *rpsF* gene promoter is located upstream of the *gsiB* gene promoter, and wherein said tandem promoter comprises the nucleotide sequence represented in the Sequence Listing as SEQ ID NO: 1.

2. A host cell of bacterium of the genus *Bacillus,* said host cell producing a target product and comprising the tandem promoter of claim 1 operably linked to at least one nucleic acid sequence that encodes a polypeptide, which is the target product, or a polypeptide required for producing the target product, wherein said tandem promoter and said at least one nucleic acid sequence operably linked to the tandem promoter are integrated into chromosome of said host cell.

3. The host cell of claim 2 selected from the group comprising the cells of *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis* and *Bacillus thuringiensis.*

4. The host cell of claim 3, wherein the host cell is *Bacillus subtilis.*

5. The host cell of claim 2, wherein at least one of the nucleic acid sequences, encoding the polypeptide that is the target product or the polypeptide required for producing the target product, is a heterologous sequence.

6. The host cell of claim 2, wherein the target product is selected from the group consisting of amino acids, polypeptides, carbohydrates, polysaccharides, nucleic acids, or derivatives thereof.

7. A method for producing a target product, said method comprising:
(a) cultivation of the host cell of any of claims 2 to 6 in a nutrient medium suitable for producing the target product, and
(b) isolation of the target product from the culture broth.

8. The method of claim 7, wherein said target product is selected from the group consisting of amino acids, polypeptides, carbohydrates, polysaccharides, nucleic acids, and derivatives thereof.
